# EUROPEAN PATENT SPECIFICATION

(11) **EP 1 992 352 B1**
(45) Date of publication and mention of the grant of the patent: **30.04.2014**
(21) Application number: 07708374.9
(22) Date of filing: 14.02.2007
(51) Int. Cl.: A23L 1/305, C07K 5/083, C07K 14/47, C07K 5/065, C07K 5/072, C07K 5/062, C07K 5/068, A61K 38/00, C12R 1/225

(54) **AGENT FOR USE IN THE PROPHYLAXIS OF ATHEROSCLEROSIS**
MITTEL ZUR VERWENDUNG IN DER PROPHYLAXE DER ATHEROSKLEROSE
AGENT POUR UNE UTILISATION DANS LA PROPHYLAXIE DE ATHEROSCLEROSIS

(30) Priority: 14.02.2006 JP 2006035945; 14.02.2006 JP 2006035946
(43) Date of publication of application: 19.11.2008
(73) Proprietor: Calpis Co., Ltd., Shibuya-ku, Tokyo 150-0022 (JP)
(72) Inventor: HIROTA, Tatsuhiko, Sagamihara-shi, Kanagawa 229-0006 (JP); NAKAMURA, Teppei, Sagamihara-shi, Kanagawa 229-0006 (JP); OHKI, Kohji, Sagamihara-shi, Kanagawa 229-0006 (JP); MASUYAMA, Akihiro, Sagamihara-shi, Kanagawa 229-0006 (JP); TAKANO, Toshiaki, Sagamihara-shi, Kanagawa 229-0006 (JP); YOSHIKAWA, Toshikazu, Uji-shi, Kyoto 611-0013 (JP); NAITO, Yuji, Rittou-shi, Shiga 520-3031 (JP); ICHIKAWA, Hiroshi, Kyoto-shi, Kyoto 606-0851 (JP); AKAGIRI, Satomi, Kyoto-shi, Kyoto 602-8032 (JP)
(74) Representative: Hallybone, Huw George
(86) International application number: PCT/JP2007/052572
(87) International publication number: WO 2007/094342

(56) References cited:
- EP-A- 0 162 032
- EP-A- 1 016 709
- WO-A-2007/132054
- WO-A1-00/41572
- WO-A1-99/16862
- JP-A- 06 040 944
- JP-A- 2003 513 636
- BONITHON-KOPP C. ET AL.: 'Plasma angiotensin-converting enzyme activity and carotid wall thickening' CIRCULATION vol. 89, no. 3, 1994, pages 952 - 954, XP003016681
- PRASAD ET AL.: 'Abnormal flow-mediated epicardial vasomotion in human coronary arteries is improved by angiotensin-converting enzyme inhibition' JOURNAL OF THE AMERICAN COLLEGE OF CARDIOLOGY vol. 33, no. 3, 1999, pages 796 - 804, XP003016680
- U. GOLDBOURT ET AL: 'Genetic aspects of arteriosclerosis' ARTERIOSCLEROSIS, THROMBOSIS, AND VASCULAR BIOLOGY vol. 6, no. 4, 01 July 1986, pages 357 - 377, XP055034095 DOI: 10.1161/01.ATV.6.4.357 ISSN: 1079-5642
- C. STOLLBERGER ET AL: 'Role of Infectious and Immune Factors in Coronary and Cerebrovascular Arteriosclerosis' CLINICAL AND VACCINE IMMUNOLOGY vol. 9, no. 2, 01 March 2002, pages 207 - 215, XP055034097 DOI: 10.1128/CDLI.9.2.207-215.2002 ISSN: 1556-6811
- F De Nigris ET AL: "Chronic treatment with sulfhydryl angiotensin-converting enzyme inhibitors reduce susceptibility of plasma LDL to in vitro oxidation, formation of oxidation-specific epitopes in the arterial wall, and atherogenesis in apolipoprotein E knockout mice", International Journal of Cardiology, vol. 81, no. 2-3, 1 December 2001 (2001-12-01), pages 107-115, XP055084344, ISSN: 0167-5273, DOI: 10.1016/S0167-5273(01)00542-3
- MARIKA SIPOLA: 'EFFECTS OF MILK PRODUCTS AND MILK PROTEIN-DERIVED PEPTIDES ONBLOOD PRESSURE AND ARTERIAL FUNCTIONIN RATS', [Online] 01 January 2002 - 01 January 2002, XP055033903 Retrieved from the Internet: <URL:http://ethesis.helsinki.fi/julkaisut/l aa/biola/vk/sipola/effectso.pdf> [retrieved on 2012-07-25]
- N. M. VAN POPELE ET AL.: "Association Between Arterial Stiffness and Atherosclerosis : The Rotterdam Study.", STROKE, vol. 32, 1 January 2001 (2001-01-01), pages 454-460, American Heart Association, 7272 Greenville Avenue, Dallas, TX 75231 USA
- YI-XIN WANG ET AL: "Increased aortic stiffness assessed by pulse wave velocity in apolipoprotein E-deficient mice", AMERICAN JOURNAL OF PHYSIOLOGY: HEART AND CIRCULATORY PHYSIOLOGY, AMERICAN PHYSIOLOGICAL SOCIETY, US, vol. 278, 1 February 2000 (2000-02-01), pages H428-H434, XP002489146, The American Physiological Society Rockville Pike Bethesda MD 20814-3991 USA ISSN: 0363-6135

## Description

The present invention relates to a prophylactic of arteriosclerosis, a vascular intimal thickening inhibitor, and a vascular endothelial function improver which have an effect of inhibiting vascular intimal thickening or improving vascular endothelial functions, and are expected to have an anti-arteriosclerotic effect, as well as functional food having such effects.

Atherosclerotic diseases, such as myocardial infarction and cerebral infarction, account for maj or part of the cause of death in Japan, along with cancers. Risk factors for arteriosclerosis include hyperlipemia, hyperlipidemia, hypertension, diabetes, smoking, obesity, hyperuricemia, aging, stress, and the like, which are interrelated to cause angiopathy. Thus even if each risk factor is low, cumulation of the factors additively and synergistically increases the risk.

There have conventionally been identified various physiological functions of fermented milk, lactic acid bacteria, and enzymatic hydrolysates of milk. For example, Patent Publications 1 and 2 report the cholesterol-lowering effect, Patent Publication 3 and Non-Patent Publication 1 report the hypotensive effect, and Patent Publication 4 reports the anti-stress effect. Patent Publication 5 describes that a casein hydrolysate, which contains free amino acids and pept ides and has been obtained by hydrolyz ing animal milk casein to have an average chain length of not longer than 2.1 in terms of the number of amino acid residues, has angiotensin I converting enzyme inhibitory activity or hypotensive effect. These publications provide information regarding mitigation of each risk factor for arteriosclerosis.

On the other hand, it is envisaged that mere mitigation of one of the above risk factors will not result inprevention of onset of arteriosclerosis. For example, Non-patent Publications 2 and 3 report the absence of interrelationship between the blood cholesterol level and onset of arteriosclerosis, Non-patent Publication 4 teaches that suppression of hypertension does not change the degree of arteriosclerosis, and Non-patent Publication 5 describes that administration of an angiotensin I converting enzyme inhibitor (enalapril) does not result in an arteriosclerosis inhibitory effect.

Accordingly, even though the particular casein hydrolysate disclosed in Patent Publication 5 is known to mitigate each risk factor for arteriosclerosis, this does not mean that the disclosed particular casein hydrolysate has an anti-arteriosclerotic effect.

An artery is mainly composed of the adventitia, the media including the smooth muscle layer which causes the blood vessel to dilate and constrict, and the intima in direct contact with blood and including the endothelial cell layer, which commands the smooth muscle layer in the media. The endothelial cells in the intima are recently being revealed to control various commands relating to vascular functions, such as fibrinolysis and coagulation of blood, dilation and constriction of blood vessels, inhibition and development of inflammation, and proliferation and regression of blood vessels. The vascular endothelial functions are believed to be associated with arteriosclerosis and various other diseases. Thus it is expected that improvement of vascular endothelial functions may prevent arteriosclerosis.

Arteriosclerosis is a pathology wherein the arterial wall is thickened to loose its elasticity. In particular, atherosclerosis is characterized by arterial subintimal spot-like thickening (atheroma), and produces symptoms of reduced or disrupted blood flow. Such symptoms are recently considered to be attributed to injury or decreased function of vascular endothelial cells.

Therefore, inhibition of vascular endothelial thickening may be expected to mitigate or prevent onset of atherosclerosis.

Diagnostic measurement of the vascular endothelial functions in human under harmless conditions is known to be performed by plethysmography, as disclosed in Non-patent Publication 6. Plethysmography is a method for determining the functions of endothelial cells through vasodilatability, utilizing the phenomenon that, when the arterial blood flow is temporarily stopped, the amount of the vasodilating substance generated and released by the endothelial cells is increased, which transiently increases the blood flow, and when the endothelial functions are impaired, the release of the vasodilating substance is lowered, which results in reduced blood flow.
Patent Publication 1: JP-2003-306436-A
Patent Publication 2: JP-2002-65203-A
Patent Publication 3: JP-2005-52090-A
Patent Publication 4: JP.-10-45610-A
Patent Publication 5: WO 2005-102542-A
Non-Patent Publication 1: American Journal of Clinical Nutrition 64 (1996), p767-771
Non-Patent Publication 2: Shoku no Kagaku 257 (1999), p20-25
Non-Patent Publication 3: Atherosclerosis 151 (2000), p501-508
Non-Patent Publication 4: Circulation 104 (2001), p2391-2394
Non-Patent Publication 5: International Journal of Cardiology 81 (2001), p107-115
Non-Patent Publication 6: The American Journal of Cardiology 87 (2001), p121-125

JP 06 040 944 A discloses how to obtain an industrially effective angiotensin converting enzyme inhibitor capable of exhibiting excellent activity inhibiting the angiotensin converting enzyme by a very small amount of oral administration, inexpensively and produced readily. The angiotensin converting enzyme inhibitor contains a peptide containing Val-Pro-Pro and having 3-10 amino acid residue number as an active ingredient. This inhibitor is produced by subjecting a food raw maternal containing a peptide composed of Val-Pro-Pro to fermentation treatment with lactic acid bacteria.

WO 99 16862 A discloses a strain of a lactic bacterium capable of producing a large amount of lactotripeptide and a fermented milk product which contains a number of components having hypotensive and stress relaxation activities and can be easily eaten and swallowed. More specifically a lactic bacterium belonging to the genus Lactobacillus helvetious, i.e. Lactobacillus helveticus strain CM4 (deposited under the accession number of FERM PB-6060 with National Institute of Bioscience and Human-Technology), which is characterized by having particular mycological properties, producing tripeptides Val-Pro-Pro and/or Ile-Pro-Pro in an amount of not less than 60 µg/ml in terms of Val-Pro-Pro when cultured in animal milk as the medium, containing not less than 9 % by weight (in terms of solid content) of non-fat milk, and exhibiting an extracellular protease activity of not less than 400 U/OD590; and a fermented milk product prepared by fermenting animal milk with the above lactic bacterium.

WO 00 41572 A1 discloses processes for producing fermented milk and milk serum whereby fermented milk and milk serum containing an ACEI peptide at a high content, which have high safety and are usable as drugs, functional foods, health foods, etc., can be efficiently obtained at a high yield. A process for preparing fermented milk containing curd pieces and milk serum containing an angiotensin converting enzyme inhibitory peptide which involves the step of mixing and stirring a milk-containing material with lactic acid bacteria to give a mixed material, and the step of fermenting the mixed material under stirring so as to form the milk serum containing the angiotensin converting enzyme inhibitory peptide; and a process for producing milk serum further involving the step of centrifuging and/or compression-filtering the fermented milk obtained above to thereby separate and collect the milk serum.

JP 2003 513636 A discloses a process for preparing a product containing antihypertensive peptides by fermenting a casein-containing starting material with lactic acid bacteria. The invention also relates to the obtained product and its use as a functional product as such or as an ingredient or additive of edible substances.

Bonithon-Kopp, et al., Circulation, 1994, 89(3), 952-954 describes a case-control study examining the relation of plasma ACE activity to intimal-medial thickness of the carotid wall measured ultrasonographically in an apparently healthy population.

Prasad, A., et al., Journal of the American College of Cardiology, 1999, 33(3), 796-804 discloses a study performed to determine whether angiotensin converting enzyme (ACE) inhibition improved endothelium-dependent flow-mediated vasodilation in patients with atherosclerosis or its risk factors and whether this is mediated by enhanced bradykinin activity.

EP-A-1 016 709 teaches a fermented milk product that contains lactic acid bacteria capable of producing a large amount of lactotripeptide and a large amount of active ingredient having hypotensive activity and anti-stress effect, and that can be taken pleasantly as foods or beverages. Lactic acid bacteria of Lactobacillus helveticus having specific bacteriological properties, the bacteria, when cultured in a medium of animal milk containing 9wt% solid of non-fat milk, producing tripeptides Val-Pro-Pro and Ile-Pro-Pro in an amount of 60 mu g in terms of Val-Pro-Pro per ml medium, and the bacteria exhibiting extracellular proteinase activity of not lower than 400U/OD590. Lactobacillus helveticus CM4 strain (deposited at National Institute of Bioscience and Human-Technology Agency of Industrial Science and Technology, deposition number FERM BP-6060).; A fermented milk product obtained by fermenting an animal milk with these lactic acid bacteria.

WO 2007/132054 A relates to preventing and treating endothelial dysfunction by using biologically active peptides and products containing them. In particular, the tripeptides Ile-Pro-Pro (IPP), Val-Pro-Pro (VPP) or mixtures, concentrates or other products containing them are used. A specific aspect of the present invention is to enhance the elasticity of blood vessels by using said biologically active peptides.

containing them are used. A specific aspect of the present invention is to enhance the elasticity of blood vessels by using said biologically active peptides.

EP-A-0 162 032 discloses a novel atrial peptide having useful natriuretic, diuretic and vasodilating activity.

It is an abject of the present invention to provide an agent according to claim 1.

It is another object of the present invention to provide the use according to claim 11.

Disclosed herein, there is provided an agent having at least one of an improving effect on vascular endothelial functions and an inhibitory effect on vascular intimal thickening, said agent comprising as an active component a hydrolysate of animal milk casein containing Xaa-Pro-Pro, or a concentrate thereof.

Disclosed herein, there is also provided an agent having at least one of an improving effect on vascular endothelial functions and an inhibitory effect on vascular intimal thickening, said agent comprising Xaa-Pro-Pro as an active component.

According to the present invention, there is also provided an agent for use in prophylaxis of atherosclerosis, said agent comprising as an active component a fermentation product containing Ile-Pro-Pro and/or Val-Pro-Pro obtained by fermenting a starting material comprising milk protein with a bacterial strain of the species *Lactobacillus helveticus.*

According to the present invention, there is provided a prophylactic of atherosclerosis comprising any of the above agents as an active component.

According to the present invention, there is also provided use of a hydrolysate of animal milk casein containing Xaa-Pro-Pro or a concentrate thereof, use of Xaa-Pro-Pro, or use of a fermentation product containing Ile-Pro-Pro and/or Val-Pro-Pro obtained by fermenting a starting material comprising milk protein with a bacterial strain of the species *Lactobacillus helveticus*, in the manufacture of a prophylactic of atherosclerosis.

Containing, as an active component, the particular casein hydrolysate or a concentrate thereof, Xaa-Pro-Pro, the particularfermentation product containing Ile-Pro-Pro and/or Val-Pro-Pro, or Xaa-Pro-Pro, the agent and the functional food according to the present invention having at least one of an improving effect on vascular endothelial functions and an inhibitory effect on vascular intimal thickening, are excellently safe, and in particular, the functional food maybe taken routinely for a prolongedperiod of time. Thus, the present agent and functional food improve chronic hypertension or moderate arteriosclerosis or vascular intimal thickening, which are associated with vascular endothelial functions, and in particular, moderate atherogenesis caused by depressed functions or injury of vascular endothelial cells due to aging or lifestyle. Consequently, mitigation or prevention of onset of atherosclerosis may be expected.

The above agent has a prophylactic effect on atherosclerosis.

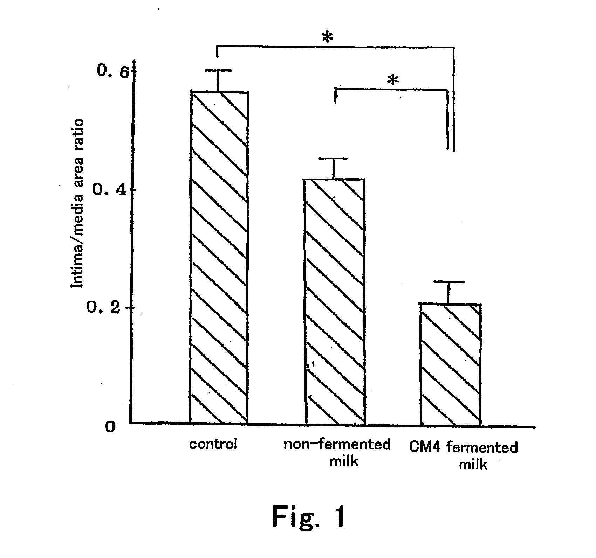

Fig. 1 is a graph showing the results of confirmatory test for the inhibitory effect on vascular intimal thickening conducted in Example 2-1 and Comparative Example 2.

The present invention will now be explained in more detail.

The agent and functional food having at least one of an improving effect on vascular endothelial functions or an inhibitory effect on vascular intimal thickening according to the present invention contain, as an active component:
(a) Xaa-Pro-Pro, which includes Ile-Pro-Pro and/or Val-Pro-Pro (referred to as component (a) hereinbelow) ;
(b) a hydrolysate of animal milk casein containing Xaa-Pro-Pro, which includes Ile-Pro-Pro and/or Val-Pro-Pro, or a concentrate thereof (referred to as component (b) hereinbelow); or
(c) a fermentation product containing Ile-Pro-Pro and/or Val-Pro-Pro obtained by fermenting a starting material containing milk protein with a bacterial strain of the species *Lactobacillus helveticus* (referred to as component (c) hereinbelow). That is, components (b) and (c) include component (a).

It is preferred that component (b) further contains Xaa-Pro.

The content of Xaa-Pro-Pro in component (b) is usually not less than 1 wt%, preferably 1 to 5 wt% of the total amount of the peptides and free amino acids in component (b). With the content of not less than 1 wt%, still more excellent effect may be expected to be achieved. Further, the contents of Ile-Pro-Pro and Val-Pro-Pro in component (b) may be independently or collectively not less than 0.3 wt% of the total amount of the peptides and free amino acids in component (b), in both of which cases excellent effect maybe expected. Further, when the contents of Ile-Pro-Pro and Val-Pro-Pro in component (b) are independently not less than 0.3 wt%, still greater effect may be expected.

The content of Xaa-Pro in component (b) is usually not less than 5 wt%, preferably 5 to 25 wt% of the total amount of the peptides and free amino acids in component (b). With the content of not less than 5 wt%, still more excellent effect may be expected.

The content of Ile-Pro-Pro and/or Val-Pro-Pro in component (c) is not less than 10 mg, preferably not less than 15 mg, per 100 g of the fermentation product in lyophilized form. With this content, the desired effect may be expected.

The Xaa in Xaa-Pro-Pro and Xaa-Pro in component (b) may be any amino acid. For example, Xaa-Pro-Pro may be Ser-Pro-Pro, Leu-Pro-Pro in addition to Ile-Pro-Pro, and/or Val-Pro-Pro, and Xaa-Pro may be Ile-Pro, Glu-Pro, Arg-Pro, Gln-Pro, Met-Pro, orTyr-Pro. Component (b) may preferably be a casein hydrolysate further containing one or two or more of the Xaa-Pro listed above.

Components (b) and (c) may contain free amino acids in addition to the peptides, and may further contain, in addition to the peptides and free amino acids, for example, lipid, ash, hydrocarbon, dietary fiber, moisture, and the like, which are usually contained in commercially available animal milk casein or milk protein. Part or all of any suitable component out of these may be removed as required.

Component (b) may be prepared, for example, by hydrolyzing animal milk casein with a group of enzymes which gives Xaa-Pro-Pro (including Ile-Pro-Pro and/or Val-Pro-Pro) and optionally Xaa-Pro as required, or by fermenting animal milk casein with koji mold.

The animal milk casein is a protein which is in foods or the like rich in Pro, and has confirmed safety. Examples of the animal milk casein may include caseins of cow' s milk, horse's milk, goat's milk, and sheep's milk, with the casein of cow's milk being preferred.

In hydrolyzing or fermenting the animal milk casein, the concentration of casein is not particularly limited, and may preferably be 3 to 19 wt% for efficient production of component (b).

The group of enzymes may preferably be Group (X) of enzymes containing, for example, a peptidase which is capable of cleaving the Pro-Xaa sequence at the carboxyl terminal of Xaa-Pro-Xaa or Xaa-Pro-Pro-Xaa.

Group (X) of enzymes may preferably include a serine proteinase having serine in its active center, or a metal proteinase having metal in its active center. The metal proteinase may be neutral protease I, neutral protease II, leucine amino peptidase, or the like. It is preferred that Group (X) of enzymes contains at least one of these metal proteinases for obtaining the desired hydrolysate efficiently in a short time in one-step reaction. The peptidase which is capable of cleaving the sequence Pro-Xaa may preferably be an enzyme having the isoelectric point in the acid region.

The group of enzymes or Group (X) of enzymes may be a group of enzymes derived from koji mold, such as *Aspergillus oryzae.* Such a group of enzymes maybe obtained by culturing the bacterial cells in a suitable medium, and extracting the produced enzymes with water. Among the groups of enzymes derived from *Aspergillus oryzae,* those having the isoelectric point in the acid region are particularly preferred.

The group of enzymes derived from *Aspergillus oryzae* may be a commercially available product, for example, Sumizyme FP, LP, or MP (all registered trademarks, manufactured by SHIN NIHON CHEMICAL CO., LTD.), Umamizyme (registered trademark, manufactured by AMANO ENZYME INC.), Sternzyme B11024, PROHIDROXY AMPL (both trade names, manufactured by HIGUCHI, INC.), Orientase ONS (registered trademark, manufactured by HANKYU BIOINDUSTRY INC.), or Denazyme AP (registered trademark, manufactured by NAGASE BIOCHEMICALS, LTD.), with Sumizyme FP (registered trademark, manufactured by SHIN NIHON CHEMICAL CO., LTD.) being particularly preferred.

For the use of these commercial products, optimal conditions are usually set, but the conditions, such as the amount of enzymes used or the reaction time, may suitably be adjusted depending on the group of enzymes to be used, so as to obtain the casein hydrolysate discussed above.

The amount of the group of enzymes use in hydrolyzing the animal milk casein may be such that, for example, the weight ratio of the group of enzymes to the animal milk casein is not lower than 1/1000, preferably 1/1000 to 1/10, more preferably 1/100 to 1/10, still more preferably 1/40 to 1/10, in an aqueous solution of the animal milk casein.

The reaction conditions may suitably be selected depending on the group of enzymes to be used, so as to obtain the objective casein hydrolysate. The temperature may usually be 25 to 60 °C, preferably 45 to 55 °C, and the pH is usually 3 to 10, preferably 5 to 9, more preferably 5 to 8. The duration of the enzymatic reaction is usually 2 to 48 hours, preferably 7 to 15 hours.

The enzymatic reaction may be terminated by inactivating the enzyme. Usually, the enzyme is inactivated at 60 to 110 °C to terminate the reaction.

After the termination of the enzymatic reaction, it is preferred to remove the resulting precipitate by centrifugation or various filtration, as desired.

Further, peptides having bitter taste or odor may be removed from the resulting hydrolysate as desired. Such bitter or odor components may be removed using activated carbon or hydrophobic resins. For example, the removal may be carried out by adding to the obtained hydrolysate 1 to 20 wt% of activated carbon with respect to the amount of casein used, and reacting for 1 to 10 hours. The activated carbon after use may be removed by a conventional method, such as centrifugation or membrane process operation.

The reaction liquid containing the resulting component (b) maybe addedas it is to a liquidproduct, such as beverages, to produce functional food. Alternatively, in order to improve the versatility of the casein hydrolysate in component (b), it is preferred to concentrate and dry the reaction liquid into a powder. By making into a powder, the reaction liquid containing component (b) may be made into a functionality imparting agent which imparts a prophylactic effect on atherosclerosis.

For improving the nutritional balance, taste, flavor, or the like, various auxiliary additives may be added to the powder, such as various hydrocarbons, lipids, vitamins, minerals, sweeteners, flavoring agents, coloring agents, or texture improvers.

The dose of the agent having the effect of improving the vascular endothelial functions and/or inhibiting vascular intimal thickening, when component (b) is the active component, is usually 10 µg to 10 g, preferably 1 mg to 5 g, more preferably about 3 mg to 1 g per day for human in terms of Xaa-Pro-Pro or the total of Xaa-Pro-Pro and Xaa-Pro in component (b) . The dose may be administered in several divided doses per day.

The dosing period may be adjusted for the symptoms of a disease, and is usually one day or longer, preferably 7 to 365 days. Regular intake is preferred.

The bacterial strain of the species *Lactobacillus helveticus* used in the preparation of component (c) is a strain of this species alone. However, in fermentation, other lactic acid bacteria may optionally be used as long as the desired effect of the present invention is not impaired.

The bacterial strain of the species *Lactobacillus helveticus* may preferably be a strain having high extracellular proteinase activity, which is preferred as bacteria capable of producing at a high yield Ile-Pro-Pro and/or Val-Pro-Pro having the effects of prophylaxis of atherosclerosis. For example, strains having a U/OD590 value of not lower than 400 are preferred, as measured in accordance with the method of Yamamoto et al. (Yamamoto N., et al., J. Biochem. (1993) 114, 740) based on the method of Twining et al. (Twining, S., Anal. Biochem. 143 3410 (1984).

A preferred example of the strain of *Lactobacillus helveticus* may be *Lactobacillus helveticus* CM4 strain (deposited at International Patent Organism Depositary, National Institute of Advanced Industrial Science and Technology, Tsukuba Central 6, 1-1-1 Higashi, Tsukuba-shi, Ibaraki, Japan, under accession number FERM BP-6060 on August 15, 1997) (referred to as CM4 strain hereinbelow) . The CM4 strain has been deposited under the above-mentioned accession number under the Budapest Treaty on the International Recognition of the Deposit of Microorganisms for the Purposes of Patent Procedure, and has already been patented.

Component (c) may be prepared by adding a fermented milk starter containing a bacterial strain of the species *Lactobacillus helveticus* to a starting material containing milk protein, and fermenting the same under suitably selected conditions, such as fermentation temperature.

Component (c) as an active component may include a powdered product prepared by, for example, lyophilizing or spray drying a concentrate of the obtained component (c).

The bacterial strain of the species *Lactobacillus helveticus* may preferably be in the form of a pre-cultured starter having sufficiently high activity. The initial cell count may preferably be about 10⁵-10⁹ cells/ml.

For use in the manufacture of, for example, functional food and beverage, such as foods for specified health uses, component (c) may be prepared by cofermentation with the strain of the species *Lactobacillus helveticus* and a yeast for giving the resulting product improved taste and flavor as well as palatability. The strain of the yeast is not particularly limited, and may preferably be, for example, yeast of the genus *Saccharomyces*, such as *Saccharomyces cerevisiae.* The content of the yeast may suitably be selected for the purpose.

The starting material containing milk protein may be, for example, animal milks, such as cow's milk, horse's milk, sheep's milk, and goat's milk; vegetable milks, such as soy bean milk; and processed milks thereof, such as skim milk, reconstituted milk, powdered milk, and condensed milk. Among these, cow's milk, soy bean milk, and processed milk thereof are preferred, and cow's milk and processed milk thereof are particularly preferred.

The solid content of the milk is not particularly limited, and when skim milk is used, the content of solid non-fat is usually about 3 to 15 wt%, and preferably 6 to 15 wt% for good productivity.

The fermentation may be carried out usually under stirring or static conditions, for example, at 25 to 45 °C, preferably 30 to 45 °C, for 3 to 72 hours, preferably 12 to 36 hours, and stopped when the lactic acid acidity reaches 1.5 % or higher.

The dose of the agent having the effect of improving vascular endothelial functions and/or inhibiting vascular intimal thickening, when component (c) is the active component, is usually 1 to 100 g, preferably about 2 to 50 g per day for human in terms of the dried product of component (c), and may be administered in several divided doses per day. The dose in terms of Val-Pro-Pro and/or Ile-Pro-Pro is usually 10 µg to 10 g, preferably 1 mg to 5 g, more preferably about 3 mg to 1 g, and may be administered in several divided doses per day.

The dosing period may be adjusted for the symptoms of a disease, and is usually one day or longer, preferably 7 to 365 days. Regular intake is preferred.

A prophylactic of arteriosclerosis disclosed herein contains an agent having the effect of improving vascular endothelial functions and/or inhibiting vascular intimal thickening. In particular, when the agent has an inhibitory effect on vascular intimal thickening, the prophylactic is useful as a prophylactic of atherosclerosis as claimed.

The dose of the prophylactic of atherosclerosis according to the present invention is usually 10 µg to 10 g, preferably 1 mg to 5 g, more preferably about 3 mg to 1 g per day for human in terms of Xaa-Pro-Pro including Ile-Pro-Pro and/or Val-Pro-Pro, the total of Xaa-Pro-Pro including Ile-Pro-Pro and/or Val-Pro-Pro and Xaa-Pro, or Val-Pro-Pro and/or Ile-Pro-Pro in components (a) to (c). The dose may be administered in several divided doses per day.

The dosing period of the prophylactic of atherosclerosis may be adjusted, taking into account the age of the human or animal to which the prophylactic is to be administered, or the environment of the human or animal associated with the risk factors of atherosclerosis. The period is usually one day or longer, preferably 7 to 365 days, and regular intake is preferred.

The prophylactic of atherosclerosis according to the present invention is usually administered orally.

The agents of the present invention may be formulated for oral administration. For example, the agents may be in the form of tablets, pills, hard capsules, soft capsules, microcapsules, powders, granules, or liquid.

The present agents may be formulated with, for example, a carrier, adjuvant, excipient, auxiliary excipient, antiseptic, stabilizer, binder, pH regulator, buffer, thickener,gelatinizer,preservative,anti-oxidant,or the like which are acceptable for pharmaceutical use, and manufactured in a unit dose form that is required in generally approved formulation.

The use of claim 11 may be to produce health foods, such as foods for specified health uses claiming the effect of prophylaxis of atherosclerosis.

The use may be to manufacture a functional food which may be taken regularly, and may be taken continuously or intermittently over a long period of time, and so the intake of the functional food for obtaining such effect is usually 10 µg to 10 g, preferably 1 mg to 5 g, more preferably about 3 mg to 1 g per day for human in terms of Xaa-Pro-Pro including Val-Pro-Pro and/or Ile-Pro-Pro or the total of Xaa-Pro-Pro including Val-Pro-Pro and/or Ile-Pro-Pro and Xaa-Pro in component (b), or of Val-Pro-Pro and/or Ile-Pro-Pro in component (c). The single intake of the functional food may be less than the above amount, depending on the number of intakes per day.

The period for taking the functional food disclosed herein is not particularly limited, and it is preferred to take it for a prolonged period of time. In order to obtain the effect discussed above, the intake period is usually one day or longer, particularly 7 to 365 days, and regular intake is preferred.

The functional food disclosed herein may optionally contain, in addition to the active component, component (b) or (c), additives, such as fermentation products with lactic acid bacteria other than *Lactobacillus helveticus,* or other components used in food and beverages, for example, sugars, proteins, lipids, vitamins, minerals, flavoring agents, and mixtures thereof.

The functional food disclosed herein may be made in any form, such as solid, gel, or liquid, by adding component (b) or (c) as it is or in powder or granular form to various food and beverage. For example, the present functional food may be in the form of fermented milk products, such as lactic acid bacteria beverages, various processed food and beverage, drypowders, tablets, capsules, granules, as well as various beverages, yogurt, fluid diet, jelly, candies, retort pouch food, tablet candies, cookies, sponge cakes, breads, biscuits, or chocolates.

The present invention will now be explained in more detail with reference to Examples, Analytic Examples, and Comparative Examples, which are illustrative only and do not limit the present invention.

### Production Example 1

1 g of casein derived from cow's milk (manufactured by NIPPON NZMP (Japan) LTD.) was added to 99 g of distilled water adjusted to about 80 °C, and the resulting mixture was thoroughly stirred. The pH of the mixture was adjusted to 7.0 with 1N sodium hydroxide solution (manufactured by WAKO PURE CHEMICAL INDUSTRIES, LTD.), and the temperature was adjusted to 20 °C, to prepare a substrate solution.

To this substrate solution, a commercially available enzyme (Sumizyme FP, registered trademark, manufactured by SHIN NIHON CHEMICAL CO., LTD.), which is derived from *Aspergillus oryzae* and contains at least metal protease, serine protease, neutral protease I, neutral protease II, and leucine amino peptidase, was added at the enzyme/casein ratio of 1/25 by weight, and the resulting mixture was reacted at 50 °C for 14 hours. Then the reaction product was autoclaved at 110 °C for 10 minutes to inactivate the enzyme, thereby obtaining a solution of enzymatic hydrolysate of casein. The solution of enzymatic hydrolysate was dried in a spray dryer to prepare a powder.

The resulting powder was analyzed for composition. The protein content was determined by the Kjeldahl method, and the amino acid content was determined with an amino acid analyzer. The peptide content was calculated by subtracting the amino acid content from the protein content. Further, the lipid content was determined by the acid hydrolysis method, the ash content by the direct ashing method, and the moisture content by the air oven method. The hydrocarbon content was taken as the remainder after subtracting the contents of these components from 100%. As a result, it was determined that the amino acid content was 35.8 wt%, the peptide content 45.7 wt%, the moisture content 6.6 wt%, the lipid content 0.2 wt%, the ash content 4.1 wt%, and the hydrocarbon content 7.6 wt%.

### <Determination of Amino Acids Constituting Peptides>

The powder prepared above was dissolved in a suitable amount of distilled water, and analyzed in an automated protein sequencer (trade name PPSQ-10, manufactured by SHIMADZU CORPORATION) for amino acid sequence from the N-terminal. Incidentally, the automatic peptide analyzer does not detect free amino acids.

The total amount of the amino acids at residue 5 was 120 pmol, and the total amount of the amino acids at residue 6 was 100 pmol. From these results, it was found that most of the peptides contained in the powder were di- and tripeptides. Further, the percentage of the peptides having Pro at residue 2 was 49.5 %, which was remarkably high, and the percentage of the peptides having Pro at residue 3 was also as high as 29.8 %.

Consequently, it is estimated that the content of Xaa-Pro or Xaa-Pro-Pro in the powder is high, and that these peptides have high resistance to enzymatic hydrolysis with proteases in the living body.

### <Determination of Peptides in Enzymatic Hydrolysate>

The powdered enzymatic hydrolysate obtained above was measured for the contents of the di- and tripeptides shown in Table 1 according to a routine method using various chemically synthesized standard peptides. The results are shown in Table 1.

**Table 1**

| Peptide Sequence | Concentration in 10 mg/ml of Powder (µg/ml) |
|---|---|
| Ile-Pro | 16.0 |
| Glu-Pro | 7.1 |
| Arg-Pro | 10.3 |
| Gln-Pro | 34.5 |
| Met-Pro | 18.4 |
| Tyr-Pro | 128.9 |
| Other Xaa-Pro | 299.4 |
| Ser-Pro-Pro | 2.9 |
| Val-Pro-Pro | 29.5 |
| Ile-Pro-Pro | 28.1 |
| Phe-Pro-Pro | 27.2 |
| Other Xaa-Pro-Pro | 28.8 |

The content of the peptides and free amino acids in a solution of the powder in distilled water was 8.15 mg/ml, the peptide content was 4.57 mg/ml, and the content of Xaa-Pro in the peptides was 514.5 µg. It was confirmed that the percentage of Xaa-Pro in the total amount of the peptides and the free amino acids in the powder was 6.3 wt%. Further, the content of Xaa-Pro-Pro in the peptides was 116.5 µg. It was confirmed that the percentage of Xaa-Pro-Pro in the total amount of the peptides and the free amino acids in the powder was 1.4 wt%.

### Example 1-1

A test for the effect of the casein hydrolysate prepared in Production Example 1 to improve vascular endothelial functions was conducted on two groups of adult males of 40 to 65 years of age having a systolic blood pressure of 140 to 159 mmHg or a diastolic blood pressure of 90 to 99 mmHg, with 24 individuals per group. The test was conducted as a double blind crossover study between the two groups, wherein 1.25 g of the powdered casein hydrolysate prepared in Production Example 1 in a capsule as a subject food or 1.25 g of sodium caseinate powder in a capsule as a placebo was given daily at or within 30 minutes after breakfast for 7 days.

In the test, the blood flow in the forearm artery was measured at rest and upon tourniquet release, before the intake of the subj ect food or placebo and after the seven-day intake, using a plethysmograph EC6, manufactured by Primetech Co. The results are shown in Table 2 as averages of each group.

**Table 2**

| | Test food | Before intake | After intake |
|---|---|---|---|
| Arterial blood flow (A) (ml/min/100ml tissue) | Subject food | 3.4±1.1 | 3.1±0.8 |
| | Placebo | 3.4±1.3 | 3.3±1.2 |
| Maximum blood flow (B) (ml/min/100ml tissue) | Subject food | 21.5±8.3 | 30.0±10.4 |
| | Placebo | 21.5±7.1 | 20.8±6.7 |
| (B)/(A)x100 | Subject food | 674.2±268.5 | 1017.0±390.3 |
| (%FBF) | Placebo | 689.3±304.5 | 673.9±266.0 |

In Table 2, each value is the average of the 24 individuals ± standard deviation, and FBF stands for forearm blood flow.

The difference in maximum blood flow and (B)/(A)x100 between the subject food group and the placebo group was significant (p<0.001). The difference in maximum blood flow and (B) / (A) x100 of the subject food group between before and after intake was significant (p<0.001).

As shown in Table 2, no significant difference in forearm arterial blood flow at rest was observed between the subj ect food group and the placebo group either before or after intake.

On the other hand, before intake, no significant difference in maximum blood flow upon tourniquet release was observed between the two groups, but after intake, the maximum blood flow was significantly higher in the subject food group, compared either to before intake or to the placebo group (both p<0.001 by t-test).

In the ratio of the maximum blood flow after reactive hyperemia to the blood flow at rest, no significant difference was observed before intake between the two groups, but the ratio was significantly higher for the subject food group after intake, compared either to before intake or to the placebo group (both p<0.001 by t-test). Incidentally, no significant improvement in blood pressure was observed during the test period.

By the results discussed above, the casein hydrolysate prepared in Production Example 1 was demonstrated to exhibit, through administration, a remarkable improving effect in the physiological evaluation of vascular endothelial functions by plethysmography, and was confirmed as an effective agent for improving vascular endothelial functions. It was also confirmed that this improving effect was not incidental to a hypotensive effect.

To the test subjects of each group, 0.3 mg of nitroglycerin was orally administered by spraying. Thereafter, the endothelium-independent vasodilator response to exogenous NO was observed over 10 minutes, and the maximum blood flow during the period was taken as the measurement. The results are shown in Table 3.

**Table 3**

| | Test Food | Before intake | After intake |
|---|---|---|---|
| Endothelium-independent vasodilator response (E) (ml/min/100ml tissue) | Subject food | 5.7±1.7 | 5.9±1.7 |
| | Placebo | 5.9±2.3 | 6.0±1.8 |
| (E) / (A) x100 | Subject food | 173.7±36.2 | 193.9±44.5 |
| (%FBF) | Placebo | 175.8±43.5 | 187.6±49.6 |

In Table 3, each value is the average of the 24 individuals in each group ± standard deviation, and FBF stands for forearm blood flow.

As shown in Table 3, no significant difference was observed in the endothelium-independent vasodilator response between before and after intake in either group, as well as between the two groups. Similar results were obtained in the ratio of the endothelium-independent vasodilator response to the blood flow at rest.

By the results discussed above, the improving effect of the casein hydrolysate prepared in Production Example 1 on the reactive blood flow was demonstrated to be independent from the improvement of the functions of the media, which responds to NO, and confirmed to be a direct reflection of the improvement in vascular endothelial functions.

### Synthesis Example

Ile-Pro-Pro and Val-Pro-Pro were synthesized through the following organic chemical synthesis by the solid phase method in an automated peptide synthesizer (PSSM-8) manufactured by SHIMADZU CORPORATION.

50 mg of 2-chlorotrityl polystyrene resin to which proline having its amino group protected with a fluorenylmethyloxycarbonyl group (abbreviated as Fmoc hereinbelow) was bound (registered trademark SynProPep resin, manufactured by SHIMADZU CORPORATION), was used as a solidphase support. 100 µmol each of Fmoc-Ile, Fmoc-Pro, and Fmoc-Val, wherein the amino groups were protected with the Fmoc group, were sequentially reacted by a routine method according to the amino acid sequence mentioned above to obtain a peptide-bound resin.

The peptide-bound resin was suspended in 1 ml of reaction liquid A (10 vol% acetic acid, 10 vol% trifluoroethanol, and 80 vol% dichloromethane), reacted at room temperature for 30 to 60 minutes to cleave the peptides from the resin, and filtered through a glass filter. The solvent in the resulting filtrate was removed under reduced pressure, and immediately 1 ml of reaction liquid B (82.5 vol% trifluoroacetic acid, 3 vol% ethyl methyl sulfide, 5 vol% purified water, 5 vol% thioanisol, 2.5 vol% ethanedithiol, and 2 vol% thiophenol) was added. The resulting mixture was reacted at room temperature for 6 hours to remove the side chain protective groups, to which 10 ml of anhydrous ether was added to precipitate the peptides. The precipitate was separated by centrifugation at 3000 rpm for 5 minutes, washed several times with anhydrous ether, and dried by spraying nitrogen gas. All of the crude synthesized peptides thus obtained was dissolved in 2 ml of a 0.1 N aqueous solution of hydrochloric acid, and purified by C18 reverse phase HPLC under the following conditions:
Pump: model L6200 intelligent pump (manufactured by HITACHI, LTD.); Detector: ultraviolet absorption at 215 nm was detected with model L4000 UV detector (manufactured by HITACHI, LTD.); Column: µBondasphere 5µ C18 (manufactured by Nihon Waters K.K.); Eluate: Liquid A of a 0.1 wt% TFA aqueous solution and Liquid B of 0.1 wt% TFA-containing acetonitrile, (B/A+B)x100 (%) : 0 to 40% (over 60 min) ; Flow rate: 1 ml/min.

The eluted fraction having the maximum absorption was taken out and lyophilized to obtain the objective synthesized peptides Ile-Pro-Pro and Val-Pro-Pro at the yields of 5.7 mg and 6.5 mg, respectively. The purified peptides were analyzed from the N-terminal in an automated protein sequencer (model PPSQ-10, manufactured by SHIMADZU CORPORATION), and further analyzed in an amino acid analyzer (model 800 series, manufactured by JASCO CORPORATION). It was confirmed that the peptides were prepared as designed.

### Example 1-2

Wistar rats (7 weeks of age, male) as the test animals were acclimatized for one week. A nitric oxide synthesis inhibitor, NG-nitro-L-arginine methyl ester hydrochloride (L-NAME, manufactured by SIGMA-ALDRICH CORP.) was dissolved in drinking water at the concentration of 1 g/L, and similarly, L-NAME and Val-Pro-Pro synthesized above at 1 g/L and 0.3 g/L, respectively, and L-NAME and Ile-Pro-Pro synthesized above at 1 g/L and 0.3 g/L, respectively. The rats were allowed free access to the prepared drinking water for one week. As a control, only the drinking water without L-NAME dissolved therein was given to a non-treatment group.

Then, the rats were subjected to fatal exsanguination under diethyl ether anesthesia. The thoracic aorta was taken out, cut into 2 mm long, and made into an aorta ring. The ring was set in a Magnus apparatus (product name "micro tissue organ bath MTOB-1Z", manufactured by LABO SUPPORT CO., LTD.) filled with 5 ml of Tyrode's solution (composition: 158.3 mM of NaCl, 4.0 mM of KCl, 2.0 mM of CaCl₂, 1.05 mM of MaCl₂, 0.42 mM of NaH₂PO₄, 10.0 mM of NaHCO₃, and 5.6 mM of glucose; pH 7.4, 37±0.5°C), and allowed to equilibrate with the resting tension of 2.0 g. The aorta ring was then allowed to constrict with 1 µM of phenylephrine. The stably constricted samples were observed for endothelium-dependent vasodilator response using 10 µM of acetylcholine. The degree of vasodilation by the response was determined as a ratio to the constriction with 1 µM of phenylephrine. The results are shown in Table 4. In the Table, eachvalue is an average of each group of 8 animals.

### Comparative Example 1

The degree of vasodilation by the vasodilation response was measured in the same way as in Example 1-2, except that the Val-Pro-Pro or Ile-Pro-Pro was replaced with an angiotensin I converting enzyme (ACE) inhibitor, enalapril, for free access at the concentration of 0.5 mg/L, which exhibited the inhibitory activity comparable to Val-Pro-Pro or Ile-Pro-Pro. The results are shown in Table 4. In the Table, each value is an average of each group of 9 animals.

**Table 4**

| Test Substance | Degree of vasodilation (%) |
|---|---|
| L-NAME | 26.1 |
| L-NAME + Val-Pro-Pro | 43.1* |
| L-NAME + Ile-Pro-Pro | 36.8* |
| L-NAME + enalapril | 31.6 |
| Non-treatment | 92.4* |

| | |
|---|---|
| *: p<0.05 | |

The results in Table 4 show that the degree of vasodilation was significantly higher in the groups given Val-Pro-Pro or Ile-Pro-Pro together with L-NAME than in the group given only L-NAME, indicating that Val-Pro-Pro and Ile-Pro-Pro improve vascular endothelial functions. On the other hand, in Comparative Example 1, wherein enalapril, which has an ACE inhibitory activity, was given, no significant difference was observed in vasodilation compared to the group given only L-NAME. It was thus demonstrated that a substance having an ACE inhibitory activity did not necessarily improve vascular endothelial functions.

### Example 2-1

### <Preparation of CM4 fermented milk feed>

A commercially available skim milk was dissolved in distilled water at 9%(w/w) solid content, subjected to high temperature pasteurization in an autoclave at 105 °C for 10 minutes, and cooled to the room temperature. Then the solution was inoculated with 3 % (v/w) of a fermentation liquid of CM4 strain starter (cell count 5 x 10⁸ cells/mL), and fermented under static conditions at 37 °C for 24 hours to obtain a CM4 fermented milk.

The CM4 fermented milk thus obtained was pasteurized at 80 °C, and lyophilized to obtain a powder. The lyophilized powder was mixed with a commercially available powder feed (trade name "CE-2", manufactured by CLEA JAPAN, INC.) at a ratio of 10 : 90 by mass to prepare a solid feed, which is referred to as a CM4 fermented milk feed. This feed contained Val-Pro-Pro and Ile-Pro-Pro derived from the CM4 fermented milk in the amounts of 34.1 mg/kg and 17.1 mg/kg, respectively. <Confirmatory Test for Effect of Inhibiting Vascular Intimal Thickening Independent of Blood Lipid Improvement>

ApoE knockout mice (5 weeks of age, male) were preliminarily bred for one week, and then allowed free access for 31 weeks to the CM4 fermented milk feed prepared above, or a solid feed as a control feedprepared from a commercially available powder feed (trade name "CE-2", manufactured by CLEA JAPAN, INC.) not containing the lyophilized powder of the CM4 fermented milk (5 animals in each group).

After the intakes, blood was collected transcardially from the mice, and subjected to biochemical tests for determining the total cholesterol, LDL cholesterol, HDL cholesterol, and triglyceride levels. The results are shown in Table 5.

The thoracic aorta of the mice was taken out and stained with hematoxylin-eosin for calculating the intima/media area ratio. The results are shown in Fig. 1 in a graph.

### Comparative Example 2

A commercially available skim milk was dissolved in distilled water at 9% (w/w) solid content, subjected to high temperature pasteurization in an autoclave at 105 °C for 10 minutes, and cooled to the room temperature. Lactic acid was added to the solution so as to give acidity comparable to that of the CM4 fermented milk prepared in Example 2-1 (2.3%), to prepare a non-fermented milk.

The non-fermented milk thus obtained was pasteurized at 80 °C, and lyophilized to obtain a powder. The lyophilized powder was mixed with a commercially available powder feed (trade name "CE-2", manufactured by CLEA JAPAN, INC.) at a ratio of 10 : 90 by mass to prepare a solid feed, which is referred to as a non-fermented milk feed. The non-fermented milk feed did not contain Xaa-Pro-Pro or Xaa-Pro.

The non-fermented milk feed was given to the mice in the same way as in Example 2-1, and the biochemical tests and calculation of vascular intima/media area ratio were carried out in the same way as in Example 2-1. The results are shown in Table 5 and Fig. 1.

**Table 5**

| | Total cholesterol (mg/dl) | | LDL cholesterol (mg/dl) | | HDL cholesterol (mg/dl) | | Triglyceride (mg/dl) | |
|---|---|---|---|---|---|---|---|---|
| | Av. | SE | Av. | SE | Av. | SE | Av. | SE |
| Control | 678.6 | 37.1 | 544.8 | 42.7 | 332.7 | 22.7 | 91.2 | 17.0 |
| Comp. Ex. 2 | 632.0 | 12.2 | 429.7 | 80.7 | 315.1 | 5.5 | 79.0 | 15.1 |
| Ex. 2-1 | 610.2 | 49.1 | 485.2 | 43.0 | 289.4 | 11.8 | 111.4 | 9.3 |

The results in Table 5 show that no improving effect on hyperlipemia was observed for the CM4 fermented milk containing Val-Pro-Pro and Ile-Pro-Pro compared to the control feed and the non-fermented milk feed. On the other hand, the results in Fig. 1 show that the CM4 fermented milk significantly inhibited vascular intimal thickening compared to the control feed and the non-fermented milk feed. Deducing from the fact that the non-fermented milk contains a comparable or even higher amount of milk components compared to the CM4 fermented milk, the effect of the present invention is not ascribable merely to milk components.

It is thus understood that the CM4 fermented milk exhibited the effect of moderating vascular intimal thickening through a mechanism different from that for improving hyperlipemia, and is thus effective against atherosclerosis caused by other than hyperlipemia of animals including human.

### Example 2-2

Val-Pro-Pro and Ile-Pro-Pro synthesized in Synthesis Example, the CM4 fermented milk prepared in Example 2-1, and the casein hydrolysate powder prepared in Production Example 1 (referred to as powder of Production Example 1 hereinbelow) were respectively mixed with a commercially available powder feed (trade name "CE-2" manufactured by CLEA JAPAN, INC.) at a ratio as shown in Table 6 to prepare solid feeds. The commercially available powder feed was also used as a control.

### <Confirmatory Test for Effect of Inhibiting Vascular Intimal Thickening>

ApoE knockout mice (6 weeks of age, male, 8 animals for each group) were allowed free access to Feeds 1 to 7 as shown in Table 6 for 31 weeks. Then the thoracic aorta of the mice was taken out, fixed in 10 % neutral buffered formalin, and subjected to the Elastica-van Gieson stain. Micrographs of the tissue specimens were scanned into the computer, and the degree of vascular intimal thickening (intima/media area ratio (%)) was calculated using an image processing software (Image J). The results are shown in Table 6. The values of the degree of thickening shown in the table are the averages of each group.

**Table 6**

| | Samples added to Feed | Amount of sample added to feed (wt%) | Val-Pro-Pro content (mg/kg feed) | Ile-Pro-Pro content (mg/kg feed) | Degree of Intimal Thickening (%) | SE |
|---|---|---|---|---|---|---|
| Feed 1 | Val-Pro-Pro | 0.0034 | 34.1 | 0.0 | 21.8 | 4.7 |
| Feed 2 | Val-Pro-Pro | 0.0340 | 341.0 | 0.0 | 18.7 | 3.2 |
| Feed 3 | Ile-Pro-Pro | 0.0017 | 0.0 | 17.1 | 25.3 | 5.4 |
| Feed 4 | Ile-Pro-Pro | 0.0170 | 0.0 | 171.0 | 20.5 | 4.6 |
| Feed 5 | CM4 fermented milk | 10.0000 | 34.1 | 17.1 | 18.5 | 5.0 |
| Feed 6 | Powder of Production Example 1 | 0.8700 | 20.9 | 24.9 | 18.7 | 4.0 |
| Feed 7 | None (control) | 0.0 | 0.0 | 0.0 | 38.0 | 6.2 |

From the results shown in Table 6, it is understood that Val-Pro-Pro and Ile-Pro-Pro inhibited vascular intimal thickening concentration-dependently. Further, the CM4 fermented milk containing Val-Pro-Pro and Ile-Pro-Pro and the powder of hydrolysate of animal milk casein produced in Production Example 1 containing Xaa-Pro-Pro and Xaa-Pro also inhibited vascular intimal thickening. It is thus understood that the agents or food containing at least one of the peptides Val-Pro-Pro and Ile-Pro-Pro moderated vascular intimal thickening, and are effective for preventing atherosclerosis.

### Examples 3-1 and 3-2 and Comparative Examples 3 and 4

Fermented milks were prepared in the same way as in Example 2-1, using CM4 strain (Example 3-1), *Lactobacillus helveticus* JCM1004 (Example 3-2), *Lactobacillus gasseri* JCM1131 (Comparative Example 3), and commercially available lyophilized starter CH-1 (manufactured by Chr. Hansen A/S, containing *Streptococcus thermophilus* and *Lactobacillus delbruckii sp. bulgaricus*)*,* respectively, under the conditions of the amount of the starter liquid added and the fermentation time as shown in Table 7, and a lyophilized product of each fermented milk was obtained.

The lyophilized product thus prepared was mixed with a commercially available powder feed (trade name "CE-2", manufactured by CLEA JAPAN, INC.) at a ratio of 10 : 90 by weight to prepare a solid feed. The Val-Pro-Pro and Ile-Pro-Pro contents of this solid feed are shown in Table 7.

The confirmatory test for the effect of inhibiting vascular intimal thickening was conducted on each of the solid feeds thus produced in the same way as in Example 2-2, and the degree of intimal thickening (intimal/media area ratio (%)) was calculated. The results are shown in Table 7. The values of the degree of intimal thickening in the table are the averages of each group.

**Table 7**

| | Amount of starter liquid added (%) | Fermentation time (hr) | Val-Pro-Pro content (mg/kg feed) | Ile-Pro-Pro content (mg/kg feed) | Degree of Intimal Thickening (%) | SE |
|---|---|---|---|---|---|---|
| Control | - | - | 0.0 | 0.0 | 28.0 | 4.5 |
| Example 3-1 | 3.0 | 24 | 33.1 | 14.1 | 13.5* | 1.5 |
| Example 3-2 | 3.0 | 24 | 16.2 | 3.5 | 11.7* | 3.1 |
| Comp. Ex. 3 | 10.0 | 48 | 0.0 | 1.9 | 25.5 | 4.4 |
| Comp. Ex. 4 | 3.0 | 24 | 6.0 | 2.4 | 19.4 | 4.2 |

| | | | | | | |
|---|---|---|---|---|---|---|
| *: p<0.05 | | | | | | |

From the results shown in Table 7, it was revealed that the products of fermentation with *Lactobacillus helveticus* moderated vascular intimal thickening, which effect was not observed for the products of fermentation with other bacterial species.

## Claims

1. An agent comprising Ile-Pro-Pro and/or Val-Pro-Pro for use in prophylaxis of atherosclerosis.

2. The agent according to claim 1, further comprising Xaa-Pro-Pro other than Ile-Pro-Pro and Val-Pro-Pro.

3. The agent according to claim 1, wherein said agent is a hydrolysate of animal milk casein, and wherein a content of said Ile-Pro-Pro and/or Val-Pro-Pro is not less than 0.3 wt % of the total amount of peptides and free amino acids in the hydrolysate of animal milk casein.

4. The agent according to claim 2, wherein said agent is a hydrolysate of animal milk casein, and wherein a content of said Ile-Pro-Pro, Val-Pro-Pro and Xaa-Pro-Pro is not less than 1 wt % of the total amount of peptides and free amino acids in the hydrolysate of animal milk casein.

5. The agent according to claim 3 or 4, wherein said hydrolysate of animal milk casein comprises Xaa-Pro selected from the group consisting of Ile-Pro, Glu-Pro, Arg-Pro, Gln-Pro, Met-Pro, Tyr-Pro, and mixture thereof.

6. The agent according to claim 5, wherein a content of said Xaa-Pro is not less than 5 wt % of the total amount of peptides and free amino acids in the hydrolysate of animal milk casein.

7. The agent according to any one of claims 3 to 6, wherein said hydrolysate of animal milk casein is a product of fermentation of animal milk casein with koji mold.

8. The agent according to any one of claims 3 to 6, wherein said hydrolysate of animal milk casein is a product of enzymatic hydrolysis of animal milk casein with an extracellular enzyme derived from *Aspergillus oryzae.*

9. The agent according to claim 1 or 2, wherein said agent is a fermentation product of a starting material comprising milk protein with a bacterial strain of the species *Lactobacillus helveticus.*

10. The agent according to claim 9, wherein said *Lactobacillus helveticus* comprises *Lactobacillus helveticus* CM4 strain (deposited at International Patent Organism Depositary, National Institute of Advanced Industrial Science and Technology under the accession number FERM BP-6060).

11. Use of an agent according to any one of claims 1 to 10, in the manufacture of a functional food for use in prophylaxis of atherosclerosis.

## Patentansprüche

1. Ein Mittel umfassend Ile-Pro-Pro und/oder Val-Pro-Pro zur Verwendung bei der Prophylaxe von Atherosklerose.

2. Das Mittel gemäß Anspruch 1, ferner umfassend Xaa-Pro-Pro, das von Ile-Pro-Pro und Val-Pro-Pro verschieden ist.

3. Das Mittel gemäß Anspruch 1, wobei das Mittel ein Hydrolysat von tierischem Milchcasein ist und wobei ein Gehalt des Ile-Pro-Pro und/oder Val-Pro-Pro nicht weniger als 0,3 Gew.-% der Gesamtmenge an Peptiden und freien Aminosäuren im Hydrolysat von tierischem Milchcasein beträgt.

4. Das Mittel gemäß Anspruch 2, wobei das Mittel ein Hydrolysat von tierischem Milchcasein ist und wobei ein Gehalt des Ile-Pro-Pro, Val-Pro-Pro und Xaa-Pro-Pro nicht weniger als 1 Gew.-% der Gesamtmenge an Peptiden und freien Aminosäuren im Hydrolysat von tierischem Milchcasein beträgt.

5. Das Mittel gemäß Anspruch 3 oder 4, wobei das Hydrolysat von tierischem Milchcasein Xaa-Pro, ausgewählt aus der Gruppe bestehend aus Ile-Pro, Glu-Pro, Arg-Pro, Gln-Pro, Met-Pro, Tyr-Pro und Gemischen davon, umfasst.

6. Das Mittel gemäß Anspruch 5, wobei ein Gehalt des Xaa-Pro nicht weniger als 5 Gew.-% der Gesamtmenge an Peptiden und freien Aminosäuren im Hydrolysat von tierischem Milchcasein beträgt.

7. Das Mittel gemäß einem der Ansprüche 3 bis 6, wobei das Hydrolysat von tierischem Milchcasein ein Produkt einer Fermentation von tierischem Milchcasein mit Koji-Schimmelpilz ist.

8. Das Mittel gemäß einem der Ansprüche 3 bis 6, wobei das Hydrolysat von tierischem Milchcasein ein Produkt einer enzymatischen Hydrolyse von tierischem Michcasein mit einem extrazellulären, von *Aspergillus oryzae* abgeleiteten Enzym ist.

9. Das Mittel gemäß Anspruch 1 oder 2, wobei das Mittel ein Fermentationsprodukt eines Milchprotein umfassenden Ausgangsmaterials mit einem Bakterienstamm der Gattung *Lactobacillus helveticus* ist.

10. Das Mittel gemäß Anspruch 9, wobei der *Lactobacillus helveticus* einen *Lactobacillus helveticus* CM4-Stamm umfasst (hinterlegt am "International Patent Organism Depositary, National Institute of Advanced Industrial Science and Technology" unter der Zugangsnummer FERM BP-6060).

11. Verwendung eines Mittels gemäß einem der Ansprüche 1 bis 10 bei der Herstellung eines funktionellen Lebensmittels zur Verwendung bei der Prophylaxe von Atherosklerose.

## Revendications

1. Agent comprenant Ile-Pro-Pro et/ou Val-Pro-Pro destiné à être utilisé dans la prophylaxie de l'athérosclérose.

2. Agent selon la revendication 1, comprenant en outre Xaa-Pro-Pro différent de Ile-Pro-Pro et Val-Pro-Pro.

3. Agent selon la revendication 1, où ledit agent est un hydrolysat de caséine de lait d'animal et où une teneur dudit Ile-Pro-Pro et/ou Val-Pro-Pro n'est pas inférieure à 0,3 % en poids de la quantité totale de peptides et d'aminoacides libres dans l'hydrolysat de caséine de lait d'animal.

4. Agent selon la revendication 2, où ledit agent est un hydrolysat de caséine de lait d'animal et où une teneur desdits Ile-Pro-Pro, Val-Pro-Pro et Xaa-Pro-Pro n'est pas inférieure à 1 % en poids de la quantité totale de peptides et d'aminoacides libres dans l'hydrolysat de caséine de lait d'animal.

5. Agent selon la revendication 3 ou 4, où ledit hydrolysat de caséine de lait d'animal comprend Xaa-Pro choisi dans le groupe consistant en Ile-Pro, Glu-Pro, Arg-Pro, Gln-Pro, Met-Pro, Tyr-Pro, et un mélange de ceux-ci.

6. Agent selon la revendication 5, où une teneur dudit Xaa-Pro n'est pas inférieure à 5 % en poids de la quantité totale de peptides et d'aminoacides libres dans l'hydrolysat de caséine de lait d'animal.

7. Agent selon l'une quelconque des revendications 3 à 6, où ledit hydrolysat de caséine de lait d'animal est un produit de fermentation de caséine de lait d'animal avec une moisissure koji.

8. Agent selon l'une quelconque des revendications 3 à 6, où ledit hydrolysat de caséine de lait d'animal est un produit d'hydrolyse enzymatique de caséine de lait d'animal avec une enzyme extracellulaire dérivée de *Aspergillus oryzae.*

9. Agent selon la revendication 1 ou 2, où ledit agent est un produit de fermentation d'une matière première comprenant des protéines du lait avec une souche bactérienne de l'espèce *Lactobacillus helveticus.*

10. Agent selon la revendication 9, où ledit *Lactobacillus helveticus* comprend la souche *Lactobacillus helveticus* CM4 (déposée auprès du International Patent Organism Depositary, National Institute of Advanced Industrial Science and Technology sous le numéro d'ordre FERM BP-6060).

11. Utilisation d'un agent selon l'une quelconque des revendications 1 à 10 dans la fabrication d'un aliment fonctionnel destiné à être utilisé dans la prophylaxie de l'athérosclérose.
